# EUROPEAN PATENT APPLICATION

(11) **EP 4 303 671 A1**
(43) Date of publication of application: **10.01.2024**
(21) Application number: 23183840.0
(22) Date of filing: 06.07.2023
(51) Int. Cl.: G05B 17/02

(54) **MODEL SELECTION APPARATUS, MODEL SELECTION METHOD, AND MODEL SELECTION PROGRAM**

(30) Priority: 08.07.2022 JP 2022110330
(71) Applicant: Yokogawa Electric Corporation, Musashino-shi, Tokyo 180-8750 (JP)
(72) Inventor: JINGUU, Yoshiyuki, Tokyo, 180-8750 (JP); OKAMOTO, Hiromi, Tokyo, 180-8750 (JP); MITOMO, Nobuhiro, Tokyo, 180-8750 (JP); TAKAMI, Go, Tokyo, 180-8750 (JP); SATO, Masahiko, Tokyo, 180-8750 (JP); FUJII, Hideyuki, Tokyo, 180-8750 (JP)
(74) Representative: Hoffmann Eitle

(57) **Abstract**

Means for solving the problem: There is provided a model selection apparatus including: an evaluation model storage unit configured to store each of a plurality of evaluation models capable of outputting an index for evaluating a state of a facility that is configured to manufacture a product from a raw material, in association with the raw material; a property data acquisition unit configured to acquire property data indicating a property of the raw material which is used in the facility; a model selection unit configured to select a target model for evaluating the state of the facility based on the property data, from among the plurality of evaluation models, when a target raw material in the facility is used; and a target model output unit configured to output the target model.

## Description

### BACKGROUND

### 1. TECHNICAL FIELD

The present invention relates to a model selection apparatus, a model selection method, and a model selection program.

### 2. RELATED ART

Patent Document 1 discloses that "the model 45 outputs a recommended control parameter indicating the first type of control content recommended for increasing the reward value in response to input of the measurement data". In addition, Non-Patent Document 1 discloses "FKDPP (Factorial Kernel Dynamic Policy Programming)".

### Prior Art Document

### Patent Document

Patent Document 1: Japanese Patent Application Publication No. 2021-086283

### Non-Patent Document

Non-Patent Document 1: "Yokogawa Electric and NAIST for Reinforcement Learning for Chemical Plants", Nikkei Robotics, March 2019

### SUMMARY

A first aspect of the present invention provides a model selection apparatus The model selection apparatus includes an evaluation model storage unit configured to store each of a plurality of evaluation models capable of outputting an index for evaluating a state of a facility that is configured to manufacture a product from a raw material, in association with the raw material; a property data acquisition unit configured to acquire property data indicating a property of the raw material which is used in the facility; a model selection unit configured to select a target model for evaluating the state of the facility based on the property data, from among the plurality of evaluation models, when a target raw material in the facility is used; and a target model output unit configured to output the target model.

In the model selection apparatus, the model selection unit may be configured to select, as the target model, an evaluation model corresponding to a raw material with a property similar to that of the target raw material, from among the plurality of evaluation models.

In any of the model selection apparatuses, the model selection unit may be configured to determine that a raw material belonging to a same cluster as that of the target raw material, as a result of clustering the property data, has a property similar to that of the target raw material.

In any of the model selection apparatuses, the model selection unit may be configured to change, when none of raw materials belongs to the same cluster as that of the target raw material as a result of hierarchical clustering of the property data, a threshold value of a distance between pieces of data, which is used for the hierarchical clustering.

In any of the model selection apparatuses, the raw material may include at least crude oil.

In any of the model selection apparatuses, the property data may have data indicating at least any of a chemical property or a physical property of the crude oil.

In any of the model selection apparatuses, the chemical property may include a content of at least any of carbon, hydrogen, sulfur, nitrogen, oxygen, or metal.

In any of the model selection apparatuses, the chemical property may include a type of a molecular structure of a hydrocarbon.

In any of the model selection apparatuses, the physical property may include at least any of specific gravity, vapor pressure, kinematic viscosity, or pour point.

In any of the model selection apparatuses, the raw material may include at least any of a fossil fuel or water.

Any of the model selection apparatuses may further include an operation model generation unit configured to generate an operation model that is configured to output an action in accordance with the state of the facility, by reinforcement learning in which an output of the target model is set as at least a part of a reward.

Any of the model selection apparatuses may further include a control unit configured to control a control target in the facility by using the operation model.

A second aspect of the present invention provides a model selection method. The model selection method is executed by a computer, and includes: by the computer, storing each of a plurality of evaluation models capable of outputting an index for evaluating a state of a facility that is configured to manufacture a product from a raw material, in association with the raw material; acquiring property data indicating a property of the raw material which is used in the facility; selecting a target model for evaluating the state of the facility based on the property data, from among the plurality of evaluation models, when a target raw material in the facility is used; and outputting the target model.

A third aspect of the present invention provides a model selection program. The model selection program is executed by a computer and causes the computer to function as: an evaluation model storage unit configured to store each of a plurality of evaluation models capable of outputting an index for evaluating a state of a facility that is configured to manufacture a product from a raw material, in association with the raw material; a property data acquisition unit configured to acquire property data indicating a property of the raw material which is used in the facility; a model selection unit configured to select a target model for evaluating the state of the facility based on the property data, from among the plurality of evaluation models, when a target raw material in the facility is used; and a target model output unit configured to output the target model.

The summary clause does not necessarily describe all necessary features of the embodiments of the present invention. The invention may also include a sub-combination of the features described above.

### BRIEF DESCRIPTION OF THE DRAWINGS

Fig. 1 shows an example of a block diagram of a model selection apparatus 100 according to the present embodiment, together with a facility 10.
Fig. 2 shows an example of a petroleum refining flow in a refinery which is an example of the facility 10.
Fig. 3 shows an example of a correspondence between a raw material and an evaluation model.
Fig. 4 shows an example of a flow diagram of a model selection method that is executed by the model selection apparatus 100 according to the present embodiment.
Fig. 5 shows examples of a data space of property data and a dendrogram, for crude oils A, B, C, and D.
Fig. 6 shows examples of a data space of property data and a dendrogram for crude oils A, B, C, D, and E.
Fig. 7 shows examples of a data space of property data and a dendrogram before threshold value changes for crude oils A, B, C, D, and F.
Fig. 8 shows examples of a data space of property data and a dendrogram after threshold value changes for crude oils A, B, C, D, and F.
Fig. 9 shows an example of a block diagram of the model selection apparatus 100 according to a first modification example of the present embodiment, together with the facility 10.
Fig. 10 shows an example of a block diagram of the model selection apparatus 100 according to a second modification example of the present embodiment, together with the facility 10.
Fig. 11 shows an example of a computer 9900 in which a plurality of aspects of the present invention may be entirely or partially embodied.

### DESCRIPTION OF EXEMPLARY EMBODIMENTS

Hereinafter, embodiments of the present invention will be described. However, the following embodiments are not for limiting the invention according to the claims. In addition, not all combinations of features described in the embodiment are essential to the solution of the invention.

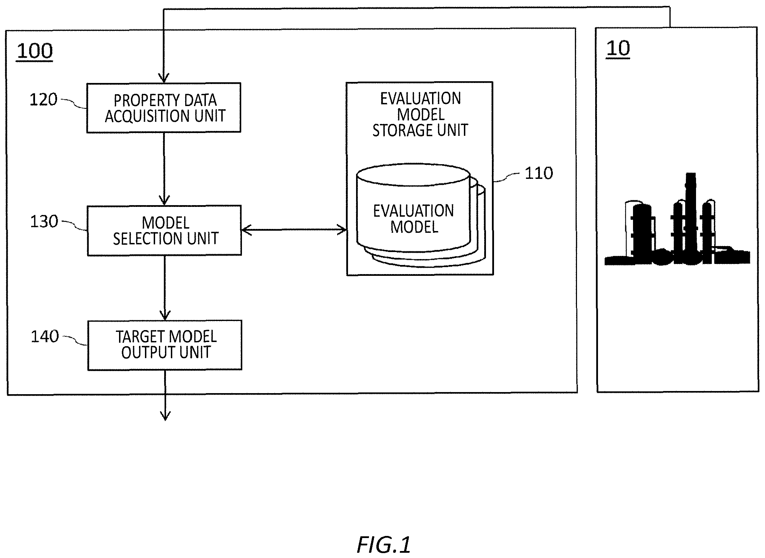

Fig. 1 shows an example of a block diagram of a model selection apparatus 100 according to the present embodiment, together with a facility 10. It should be noted that these blocks are functional blocks which are each functionally divided, and may not be necessarily required to be matched with actual apparatus configurations. That is, in the present drawing, an apparatus indicated by one block may not be necessarily required to be configured by one apparatus. In addition, in the present drawing, apparatuses indicated by separate blocks may not be necessarily required to be configured by separate apparatuses. The same applies to subsequent block diagrams.

The facility 10 is an apparatus (a group of apparatuses) that manufactures a product from a raw material. For example, the facility 10 may be a plant or a complex apparatus in which a plurality of pieces of equipment are combined. Examples of the plant include: in addition to an industrial plant such as a chemical plant and a biotechnology plant, a plant for managing and controlling a well site such as a gas field or an oil field and its surrounding area; a plant for managing and controlling power generation such as hydroelectric, thermal, or nuclear power generation; a plant for managing and controlling energy harvesting from solar power, wind power, or the like; a plant for managing and controlling water and sewerage, dams, or the like; and others.

The facility 10 may be provided with various control targets, and may also be provided with one or more sensors capable of measuring various states (physical quantities) inside and outside the facility 10. As an example, such a sensor may output process variables PVs (Process Variable) obtained by measuring temperatures at various positions of the facility 10, flow rates in various paths, or the like. State data indicating a state of the facility 10 may include such a process variable PV. The state data may also include a manipulated variable MV (Manipulated Variable) indicating a degree of opening and closing of a control target (for example, a valve) or the like. In addition to operation data indicating an operation state as a result of controlling the control target in this way, the state data may include consumption amount data indicating an amount of consumption of energy or the raw material in the facility 10, and disturbance environment data or the like indicating the physical quantity that can act as a disturbance on the control of the control target.

Here, in operating the facility 10, it is desired to appropriately evaluate the state of the facility 10 in light of an operation target. In evaluating the state of the facility 10, it is conceivable to use an evaluation model that is trained by machine learning to output an index for evaluating the state of the facility 10, according to inputting the state data indicating the state of the facility 10.

For example, the facility 10 is set to be a refinery that refines crude oil to manufacture (produce) a plurality of petroleum products. In this case, the raw material which is used in the facility 10 includes at least crude oil. However, it is known that the crude oil has a great difference in property depending on a production region or a production time. It should be noted that the "property" may be interpreted to mean at least any of a natural characteristic or a state of an object. Such a difference in property causes differences in reactivity, corrosiveness, toxicity, and the like, and also causes differences in change of a product suitable for manufacturing, a product quality, and the like.

For the operation target of the facility 10, a plurality of items, such as quality assurance, energy saving, GHG (Green House Gas) reduction, and yield enhancement, are able to be set, and various target values are able to be set for each item. It is ideal for such an operation target to be optimized for each crude oil. That is, it is ideal to generate the evaluation model for each crude oil. However, when a new evaluation model is generated every time the production region or the production time of the crude oil is changed, a processing load increases due to an increase in frequency of the machine learning, and a time loss occurs because it is necessary to newly generate the evaluation model before an operation of the facility 10 is started.

Therefore, the model selection apparatus 100 according to the present embodiment stores a plurality of evaluation models in association with raw materials, and selects a target model for evaluating the state of the facility 10 based on property data indicating the property of the raw material, from among the plurality of evaluation models.

As an example, a case where the facility 10 is a refinery will be described below. However, the present invention is not limited to this. The facility 10 may be any apparatus (a group of apparatuses) capable of manufacturing the product from the raw material, and in particular, an apparatus (a group of apparatuses) in which the property of the raw material has a comparatively great influence on the operation, is preferable. Examples of such a facility 10 include, for example, a coal-fired power plant in which a sulfur content of coal may greatly affect a degree of combustion, a water purification plant in which a liquid quality of raw water may greatly affect an amount of chemicals to be used, or the like. For example, when the facility 10 is a thermal power plant, the raw material may include a fossil fuel (such as petroleum, coal, and natural gas) or water, and the product may be electricity. In addition, for example, when the facility 10 is the water purification plant, the raw material may include at least raw water (such as river water), and the product may be purified water. In this way, the facility 10 is not limited to any of an industry type, a business scale, or the like.

The model selection apparatus 100 may be a computer such as a PC (a personal computer), a tablet type computer, a smartphone, a workstation, a server computer, or a general purpose computer, or may be a computer system in which a plurality of computers are connected. Such a computer system is also a computer in a broad sense. In addition, the model selection apparatus 100 may be implemented by one or more virtual computer environments which are able to be run in the computer. Instead of this, the model selection apparatus 100 may be a dedicated computer designed to select a model, or may be dedicated hardware realized by dedicated circuitry. In addition, in a case where a connection to the Internet is possible, the model selection apparatus 100 may be realized by cloud computing.

The model selection apparatus 100 includes an evaluation model storage unit 110, a property data acquisition unit 120, a model selection unit 130, and a target model output unit 140.

The evaluation model storage unit 110 is configured to store each of a plurality of evaluation models capable of outputting an index for evaluating the state of the facility 10 that is configured to manufacture the product from the raw material, in association with the raw material. More specifically, the evaluation model storage unit 110 may store the evaluation model for each of a plurality of clusters obtained by clustering the raw materials. This will be described below in detail. Such an evaluation model may be, as an example, a machine learning model generated by the machine learning. The evaluation model storage unit 110 may store, for example, the evaluation model generated by the machine learning in its own apparatus, or may store the evaluation model generated by the machine learning in another apparatus.

The property data acquisition unit 120 is configured to acquire the property data indicating the property of the raw material which is used in the facility 10. For example, the property data acquisition unit 120 may acquire such property data from the facility 10 via a network. However, the present invention is not limited to this. The property data acquisition unit 120 may acquire the property data via other means such as a user input or various memory devices, or may acquire the property data from another apparatus different from the facility 10. The property data acquisition unit 120 supplies the acquired property data to the model selection unit 130.

The model selection unit 130 is configured to select the target model for evaluating the state of the facility 10 based on the property data, from among the plurality of evaluation models, when a target raw material in the facility 10 is used. For example, the model selection unit 130 may refer to the property data of the raw material which is stored in the evaluation model storage unit 110, and select the target model based on the property data acquired by the property data acquisition unit 120. The model selection unit 130 reads the evaluation model selected as the target model from the evaluation model storage unit 110, and supplies the read evaluation model to the target model output unit 140.

The target model output unit 140 is configured to output the target model. For example, the target model output unit 140 may output the target model selected by the model selection unit 130 by transmitting the target model to another apparatus via the network. However, the present invention is not limited to this. The target model output unit 140 may output the selected target model by writing the selected target model to various memory devices, or may output the selected target model by displaying the selected target model on a monitor.

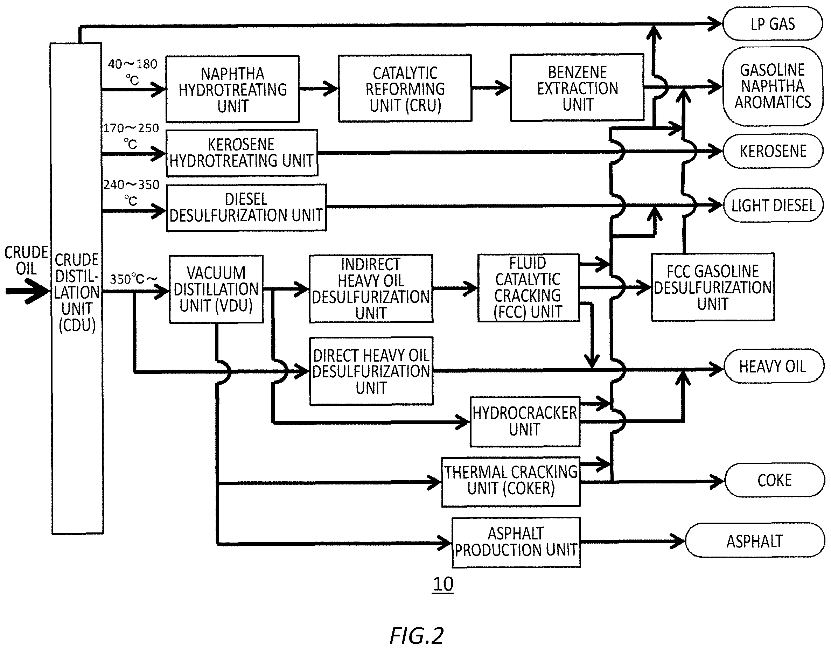

Fig. 2 shows an example of a petroleum refining flow in a refinery which is an example of the facility 10. The refinery refines the crude oil which is a mixture of hydrocarbons with a wide boiling range, to produce a plurality of petroleum products. Typically, in the refinery, the crude oil is distilled by a crude distillation unit (CDU), and is separated into fractions with boiling point ranges different from each other depending on cut temperatures, that is, a gas fraction, a naphtha fraction, a kerosene fraction, a light diesel oil fraction, a heavy diesel oil fraction, and a residue. LP gas is produced from the gas fraction. The naphtha fraction is hydro-desulfurized by a naphtha hydrotreating unit and then catalytically reformed by a catalytic reforming unit (CRU), and benzene is separated therefrom by a benzene extraction unit to produce gasoline, naphtha, aromatics, and the like. The kerosene fraction is hydro-desulfurized by a kerosene hydrotreating unit to produce kerosene. The light diesel oil fraction is desulfurized by a diesel desulfurization unit to produce light oil. The heavy diesel oil fraction is hydro-desulfurized by a direct heavy oil desulfurization unit to produce heavy oil. Also, the heavy diesel oil fraction is separated into light and heavy fractions by a vacuum distillation unit (VDU). The light fraction separated by the VDU is hydro-desulfurized by an indirect heavy oil desulfurization unit, and then catalytically cracked by a fluid catalytic cracking (FCC) unit, and is hydro-desulfurized by an FCC gasoline desulfurization unit to produce gasoline. Alternatively, the light fraction separated by the VDU is processed by a hydrocracker unit (HCU). On the other hand, the heavy fraction separated by the VDU is pyrolyzed by a thermal cracking unit (Coker) to produce coke, and is also processed by an asphalt production unit to produce asphalt.

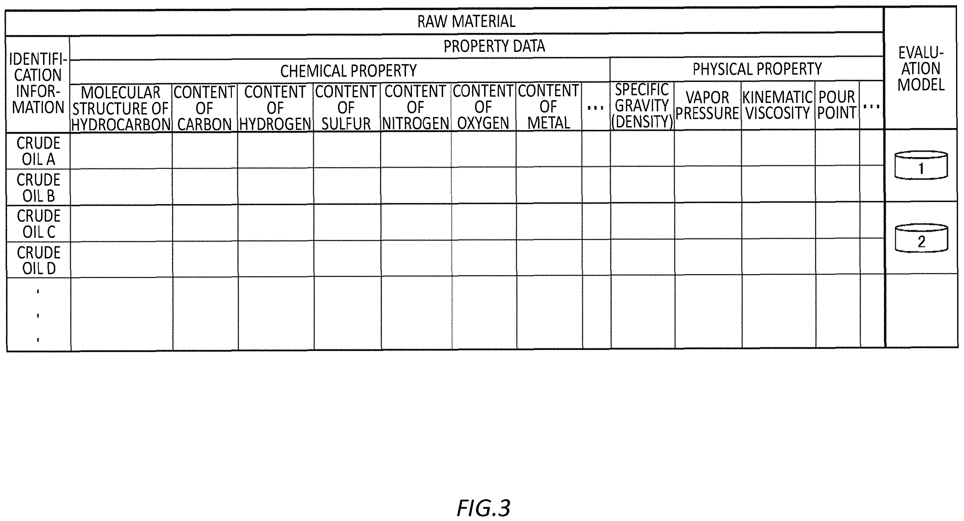

Fig. 3 shows an example of a correspondence between a raw material and an evaluation model. In the present drawing, a case where the raw material includes at least the crude oil is shown as an example. However, the present invention is not limited to this, and the raw material may include, for example, at least any of the fossil fuel or the water. The evaluation model storage unit 110 may store the plurality of evaluation models in association with data indicating the raw materials which are used in the facility 10, for example, as shown in the present drawing.

The data indicating the raw material may be constituted by, for example, identification information for identifying the raw material and the property data indicating the property of the raw material.

In the present drawing, as an example, four pieces of identification information of crude oil A, crude oil B, crude oil C, and crude oil D are shown as the identification information for identifying the raw material. Here, the crude oil identified by the crude oil A, the crude oil B, the crude oil C, and the crude oil D may be crude oil used in the facility 10 in the past, and for example, may be different from each other in at least any of the production region or the production time.

The property data indicating the property of the raw material may have data indicating at least any of a chemical property or a physical property of the crude oil. In the present drawing, a case in which the property data has both of the chemical property and the physical property is shown as an example.

The crude oil is a natural product of which a main component is set as a hydrocarbon. Depending on a type of a molecular structure of the hydrocarbon that is the main component of the crude oil, the crude oil is classified into various types, such as: crude oil containing a large amount of paraffinic hydrocarbons (referred to as "paraffin base crude oil"); crude oil containing a large amount of naphthenic hydrocarbons (referred to as "naphthenic base crude oil"); intermediate crude oil between the paraffin base crude oil and the naphthenic base crude oil (referred to as "mixed base crude oil"); and crude oil containing a large amount of aromatic hydrocarbons (referred to as "special crude oil").

Here, depending on the type of the molecular structure of the hydrocarbon, products suitable for production are different from each other, as the paraffin base crude oil for lubrication oil or wax; the naphthenic base crude oil for gasoline or asphalt; mixed base crude oil for kerosene, lubrication oil, or heavy oil; the special crude oil for gasoline or a solvent; and others. Accordingly, it is particularly preferable for the chemical property to include the type of the molecular structure of the hydrocarbon.

In addition, the crude oil contains a small amount of sulfur, nitrogen, oxygen, metal, and the like, in addition to the hydrocarbon which is the main component. A range of a typical composition (% by mass) of the crude oil is that 83% to 87% is carbon, 11% to 14% is hydrogen, 5% or less is sulfur, 0.4% or less is nitrogen, 0.5% or less is oxygen, and 0.5% or less is metal.

Here, sulfur, nitrogen, oxygen, and metal are quantitatively smaller than carbon and hydrogen, but have great influences on the petroleum refining and a quality of the petroleum product. For example, by burning, sulfur may become sulfurous acid gas which may cause deterioration of the petroleum product, corrosion of an apparatus, catalyst poisoning, and the like. In addition, metal (in particular, heavy metal) may cause the catalyst poisoning of the plant. Accordingly, it is particularly preferable for the chemical property to include a content of at least any of carbon, hydrogen, sulfur, nitrogen, oxygen, or metal.

In addition, depending on the specific gravity, the crude oil is classified into extra light crude oil, light crude oil, medium-grade crude oil, heavy crude oil, extra heavy crude oil, and the like. It should be noted that the term "specific gravity" as used herein may be interpreted to also include a "density" having an equivalent value. In addition, vapor pressure, kinematic viscosity, pour point, and the like are important items from a viewpoint of a storage or a control of the crude oil. Accordingly, it is particularly preferable for the physical property to include at least any of the specific gravity, the vapor pressure, the kinematic viscosity, or the pour point.

As an example, the present drawing shows, based on the property data indicating such a chemical property or a physical property, a case where the crude oil A and the crude oil B are clustered in the same cluster 1, and the crude oil C and the crude oil D are clustered in the same cluster 2, and then, the cluster 1 is associated with an evaluation model 1, and the cluster 2 is associated with an evaluation model 2. This means that the evaluation model 1 is used to evaluate the state of the facility 10 when the crude oil A or the crude oil B is used in the facility 10 and that the evaluation model 2 is used to evaluate the state of the facility 10 when the crude oil C or the crude oil D is used in the facility 10.

For example, the model selection apparatus 100 according to the present embodiment stores the plurality of evaluation models in association with the raw materials in this way, and when crude oil E or crude oil F is used as the target raw material in the facility 10, the model selection apparatus 100 selects the target model for evaluating the state of the facility 10, based on the property data of the crude oil, from among the plurality of evaluation models. This will be described in detail by using a flow.

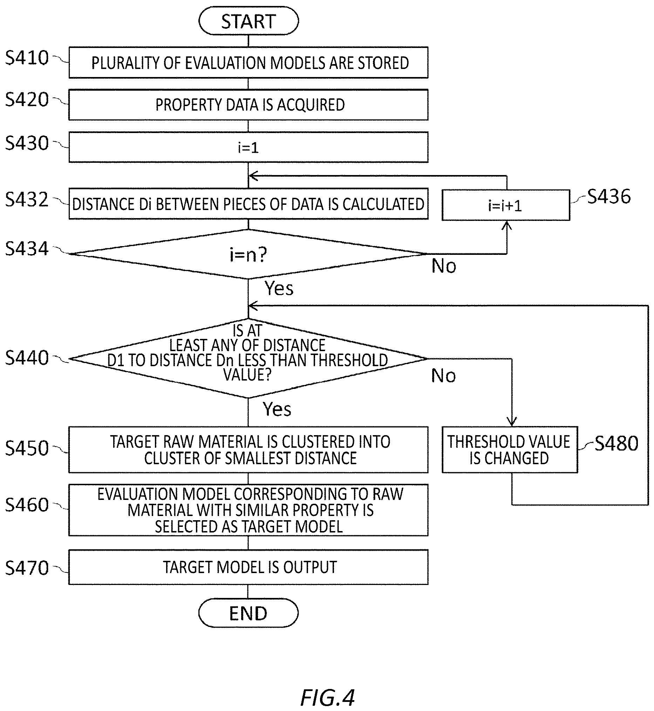

Fig. 4 shows an example of a flow diagram of a model selection method that is executed by the model selection apparatus 100 according to the present embodiment.

In step S410, the model selection apparatus 100 stores the plurality of evaluation models. For example, the evaluation model storage unit 110 may store each of the plurality of evaluation models capable of outputting the index for evaluating the state of the facility 10 that manufactures the product from the raw material, in association with the raw material. As an example, the evaluation model storage unit 110 may store the evaluation model for each of a plurality of clusters obtained by clustering the raw materials. Here, the evaluation model storage unit 110 is set to store the evaluation model 1 corresponding to the cluster 1, and the evaluation model 2 corresponding to the cluster 2, for example, as shown in Fig. 3.

Here, the evaluation model may be the machine learning model generated by the machine learning. As an example, an evaluation model generation apparatus may acquire an operation target (a plant KPI (Key Performance Indicator: key performance evaluation index) or the like) for the facility 10, the state data indicating the state of the facility 10, and a teacher label, and generate labeling data based on these. Then, the evaluation model generation apparatus may set the generated labeling data as learning data, and generate the evaluation model by an algorithm of the machine learning. For a generation process itself of the evaluation model, any process may be used, and thus, a further description will be omitted here. It should be noted that such an evaluation model generation apparatus may be the evaluation model selection apparatus 100 itself, or may be another apparatus different from the evaluation model selection apparatus 100. Accordingly, the evaluation model storage unit 110 may store, for example, the evaluation model generated by the machine learning in its own apparatus, or may store the evaluation model generated by the machine learning in another apparatus.

In step S420, the model selection apparatus 100 acquires the property data. For example, the property data acquisition unit 120 may acquire the property data indicating the property of the raw material which is used in the facility 10, from the facility 10 via the network. As an example, when the crude oil E is used as the raw material in the facility 10, the property data acquisition unit 120 may acquire property data Pe indicating the property of the crude oil E. Similarly, when the crude oil F is used as the raw material in the facility 10, the property data acquisition unit 120 may acquire property data Pf indicating the property of the crude oil F.

As described above, such property data indicating the property of the raw material may have data indicating at least any of the chemical property or the physical property of the crude oil. In addition, it is particularly preferable for the chemical property to include the type of the molecular structure of the hydrocarbon, or the content of at least any of carbon, hydrogen, sulfur, nitrogen, oxygen, or metal. In addition, it is particularly preferable for the physical property to include at least any of the specific gravity, the vapor pressure, the kinematic viscosity, or the pour point. Here, the property data is set to have data indicating all of the items described above. The property data acquisition unit 120 supplies the acquired property data to the model selection unit 130.

In step S430, the model selection apparatus 100 sets i=1. Here, i indicates an index of the cluster. In this manner, the model selection apparatus 100 initializes the index of the cluster.

In step S432, the model selection apparatus 100 calculates a distance Di between pieces of data. For example, the model selection unit 130 may calculate the distance Di between pieces of data, between the property data acquired in step S420, and the cluster i. At this time, as an example, the model selection unit 130 may calculate the distance Di, by averaging the distances between the property data acquired in step S420 and the property data of all of the raw materials clustered into the cluster i.

That is, when i=1, the model selection unit 130 may calculate a distance Dae between pieces of data, between the property data Pe indicating the property of the crude oil E and property data Pa indicating the property of the crude oil A clustered in the cluster 1, and a distance Dbe between pieces of data, between the property data Pe indicating the property of the crude oil E and property data Pb indicating the property of the crude oil B clustered in the cluster 1, respectively; and average the distance Dae and the distance Dbe to calculate a distance D1e between pieces of data, between the property data Pe and the cluster 1.

Similarly, the model selection unit 130 may calculate a distance Daf between pieces of data, between the property data Pf indicating the property of the crude oil F and the property data Pa indicating the property of the crude oil A clustered in the cluster 1, and a distance Dbf between pieces of data, between the property data Pf indicating the property of the crude oil F and the property data Pb indicating the property of the crude oil B clustered in the cluster 1, respectively; and average the distance Daf and the distance Dbf to calculate a distance D1f between pieces of data, between the property data Pf and the cluster 1.

It should be noted that in the above description, the case where the model selection unit 130 calculates the distance Di by using a group average method is shown as an example; however, the present invention is not limited to this. The model selection unit 130 may calculate the distance Di by using another method such as Ward's method.

In step S434, the model selection apparatus 100 determines whether i=n. Here, n indicates a maximum value of the index of the cluster. For example, when the evaluation model storage unit 110 stores the evaluation model 1 corresponding to the cluster 1 and the evaluation model 2 corresponding to the cluster 2, n=2. When i=1, i=n is not true (No), and thus, the model selection apparatus 100 advances processing to step S436.

In step S436, the model selection apparatus 100 sets i=i+1. In this manner, the model selection apparatus 100 increments the index of the cluster. Then, the model selection apparatus 100 returns the processing to step S432 and continues the flow.

In step S432, when i=2, the model selection unit 130 may calculate a distance Dce between pieces of data, between the property data Pe indicating the property of the crude oil E and property data Pc indicating the property of the crude oil C clustered in the cluster 2, and a distance Dde between pieces of data, between the property data Pe indicating the property of the crude oil E and property data Pd indicating the property of the crude oil D clustered in the cluster 2, respectively; and average the distance Dce and the distance Dde to calculate a distance D2e between pieces of data, between the property data Pe and the cluster 2.

Similarly, the model selection unit 130 may calculate a distance Dcf between pieces of data, between the property data Pf indicating the property of the crude oil F and the property data Pc indicating the property of the crude oil C clustered in the cluster 2, and a distance Ddf between pieces of data, between the property data Pf indicating the property of the crude oil F and the property data Pd indicating the property of the crude oil D clustered in the cluster 2, respectively; and average the distance Dcf and the distance Ddf to calculate a distance D2f between pieces of data, between the property data Pf and the cluster 2.

In step S434, when i=2, i=n (Yes), and thus, the model selection apparatus 100 advances the processing to step S440.

In step S440, the model selection apparatus 100 determines whether at least any of the distance D1 to the distance Dn is less than a threshold value. Then, if at least any of the distance D1 to the distance Dn is less than the threshold value (Yes), the model selection apparatus 100 advances the processing to step S450. For example, when the target raw material is the crude oil E, the model selection apparatus 100 may compare each of the distance D1e and the distance D2e with a predetermined threshold value. Then, if at least any of the distance D1e or the distance D2e is less than the threshold value (Yes), the model selection unit 130 may advance the processing to step S450.

In step S450, the model selection apparatus 100 clusters the target raw material into the cluster of a smallest distance. For example, when the distance D1e>the distance D2e, the model selection unit 130 may cluster the crude oil E into the cluster 2.

In step S460, the model selection apparatus 100 selects, as the target model, the evaluation model corresponding to the raw material with a similar property. As an example, when the crude oil E is clustered into the cluster 2 in step S450, the model selection unit 130 may determine that the crude oil C and the crude oil D belonging to the cluster 2 have properties similar to that of the crude oil E. Then, the model selection unit 130 may select, as the target model, the evaluation model 2 corresponding to the crude oil C and the crude oil D, that is, the cluster 2. The model selection unit 130 may determine that the raw material belonging to the same cluster as that of the target raw material, as a result of clustering the property data, for example, in this way, is similar to the target raw material. Then, the model selection unit 130 may select, as the target model, the evaluation model corresponding to the raw material with the property similar to that of the target raw material, from among the plurality of evaluation models. It should be noted that being "similar" may be interpreted to include being the same in addition to being similar.

In step S470, the model selection apparatus 100 outputs the target model. For example, the model selection unit 130 may read, from the evaluation model storage unit 110, the evaluation model selected as the target model in step S460, and supply the read evaluation model to the target model output unit 140. Then, the target model output unit 140 may transmit the target model to another apparatus via the network.

Here, a technique in which a control apparatus controls the control target in the facility 10 by using an operation model that outputs an action in accordance with the state of the facility 10, is known as a so-called AI (Artificial Intelligence) control. In addition, it is conceivable that the operation model generation apparatus generates the operation model which is used for the AI control, by reinforcement learning in which the output of the evaluation model is set as at least a part of a reward. Accordingly, the target model output unit 140 may transmit the selected target model to such an operation model generation apparatus. According to this, the operation model generation apparatus may generate the operation model that outputs the action in accordance with the state of the facility 10, by the reinforcement learning in which the output of the evaluation model selected as the target model is set as at least a part of the reward. Then, the operation model generation apparatus may transmit the generated operation model to the control apparatus. According to this, the control apparatus may perform the control, that is, the AI control on the control target provided in the facility 10 by using the operation model.

It should be noted that in the above description, the case where the model selection apparatus 100 outputs the selected target model itself, is shown as an example; however, the present invention is not limited to this. For example, the model selection apparatus 100 may output information identifying the selected target model. In this case, the model selection unit 130 may supply, to the target model output unit 140, the information identifying the evaluation model selected as the target model, and the target model output unit 140 may output this. In this way, the term "outputting the model" as used herein may be broadly interpreted to include outputting the information identifying the model in addition to outputting the model itself.

On the other hand, if none of the distance D1 to the distance Dn is less than the threshold value (No) in step S440, the model selection apparatus 100 advances the processing to step S480. For example, when the target raw material is the crude oil F, the model selection unit 130 may compare each of the distance D1 and the distance D2 with a predetermined threshold value. If none of the distance D1f and the distance D2f is less than the threshold value (No), the model selection unit 130 may advance the processing to step S480.

In step S480, the model selection apparatus 100 changes the threshold value. For example, the model selection unit 130 may change the threshold value by increasing the threshold value which is used in step S440 by a predetermined change width Δ. When none of the raw materials belongs to the same cluster as that of the target raw material as a result of hierarchical clustering of the property data, for example, in this way, the model selection unit 130 may change the threshold value of the distance between pieces of data, which is used for the hierarchical clustering. Then, the model selection apparatus 100 returns the processing to step S440 and continues the flow. Accordingly, the model selection apparatus 100 may change the threshold value in a stepwise manner until at least any of the distance D1 to the distance Dn becomes less than the threshold value.

As an example, the distance D2f is set to be less than the threshold value in step S440 as a result of increasing the threshold value by the change width Δ in step S480. In this case, the model selection unit 130 advances the processing to step S450. Then, the model selection unit 130 groups the crude oil F into the cluster 2 in step S450, and selects the evaluation model 2 corresponding to the cluster 2 as the target model in step S460. According to this, the target model output unit 140 outputs the selected target model in step S470.

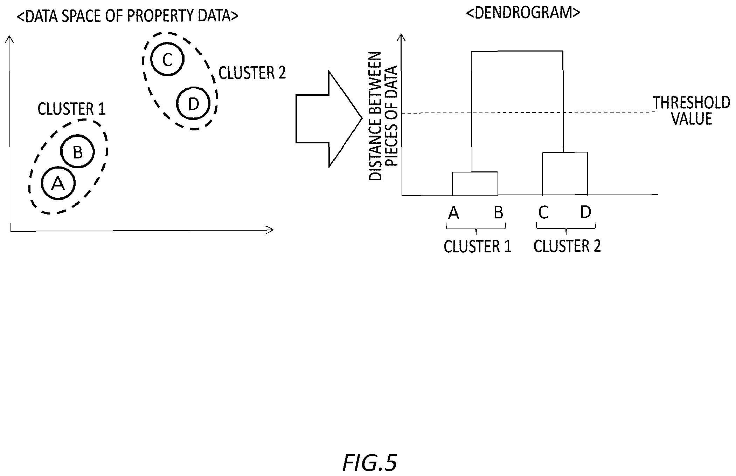

Fig. 5 shows examples of a data space of property data and a dendrogram, for crude oils A, B, C, and D. An example of using the hierarchical clustering when the model selection apparatus 100 clusters the property data will be described below. In a case where the hierarchical clustering is used, it is preferable because the processing is comparatively simple and the classification process can be clarified. However, the present invention is not limited to this. In clustering the property data, the model selection apparatus 100 may use non-hierarchical clustering such as a k-means method, a k-means++ method, and an x-means method.

A left part of the present drawing shows the data space of the property data. As shown in the left part of the present drawing, the crude oil A and crude oil B are clustered in the cluster 1, and the crude oil C and crude oil D are clustered in the cluster 2.

A right part of the present drawing shows the dendrogram. A dendrogram is a tree diagram representing how individuals are respectively grouped into the clusters in the clustering process. According to the dendrogram, it can be seen that: the crude oil A and the crude oil B are clustered in the same cluster because the distance between pieces of data is short (less than the threshold value); the crude oil C and the crude oil D are clustered in the same cluster because the distance between pieces of data is short; and on the other hand, the crude oil A and the crude oil B, and the crude oil C and the crude oil D are clustered in the clusters different from each other because the distance between pieces of data is long (greater than or equal to the threshold value). In clustering the property data, the model selection unit 130 may visualize the process in which the cluster is formed by outputting (for example, displaying on a monitor) such a dendrogram.

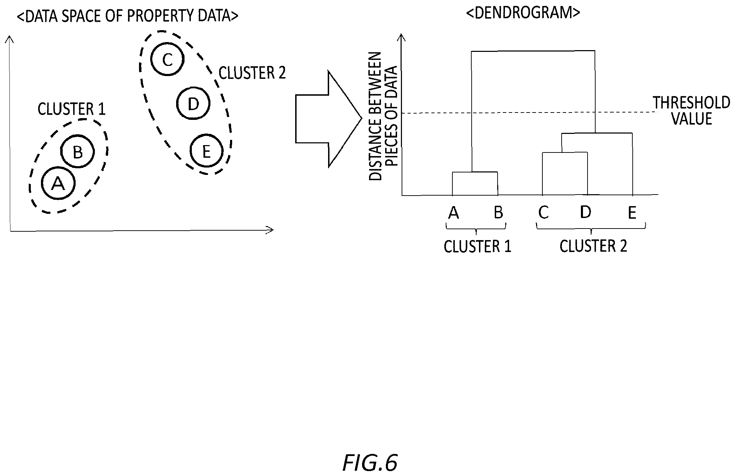

Fig. 6 shows examples of a data space of property data and a dendrogram for crude oils A, B, C, D, and E. In Fig. 6, members having the same functions and configurations as those in Fig. 5 are denoted by the same signs and numerals, and the descriptions thereof will be omitted except for the following differences.

According to the dendrogram, it can be seen that: the distance between pieces of data, between the crude oil E and the cluster 1 is long (greater than or equal to the threshold value); on the other hand, the distance between pieces of data, between the crude oil E and the cluster 2 is short (less than the threshold value); and thus, the crude oil E is clustered in the cluster 2. In this case, the model selection unit 130 may determine that the crude oil C and the crude oil D belonging to the cluster 2 have properties similar to that of the crude oil E. Then, the model selection unit 130 may select, as the target model when the crude oil E is used in the facility 10, the evaluation model 2 corresponding to the crude oil C and the crude oil D, that is, the cluster 2. According to this, the target model output unit 140 may output the evaluation model 2 as the target model.

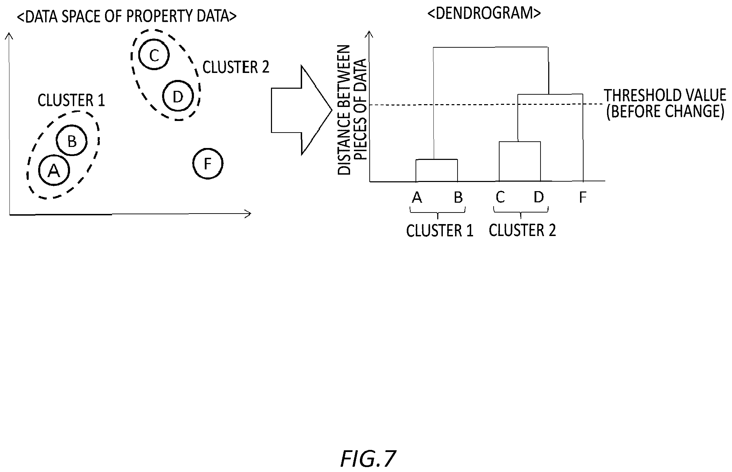

Fig. 7 shows examples of a data space of property data and a dendrogram before threshold value changes for crude oils A, B, C, D, and F. In Fig. 7, members having the same functions and configurations as those in Fig. 5 are denoted by the same signs and numerals, and the descriptions thereof will be omitted except for the following differences.

According to the dendrogram, it can be seen that: the distance between pieces of data, between the crude oil F and the cluster 1 is long (greater than or equal to the threshold value); and the distance between pieces of data, between the crude oil F and the cluster 2 is long; and thus, the crude oil F is not clustered in any of the cluster 1 and the cluster 2. In this case, the model selection unit 130 cannot find crude oil with a property similar to that of the crude oil F. Therefore, the model selection unit 130 may change the threshold value of the distance between pieces of data, which is used for the hierarchical clustering.

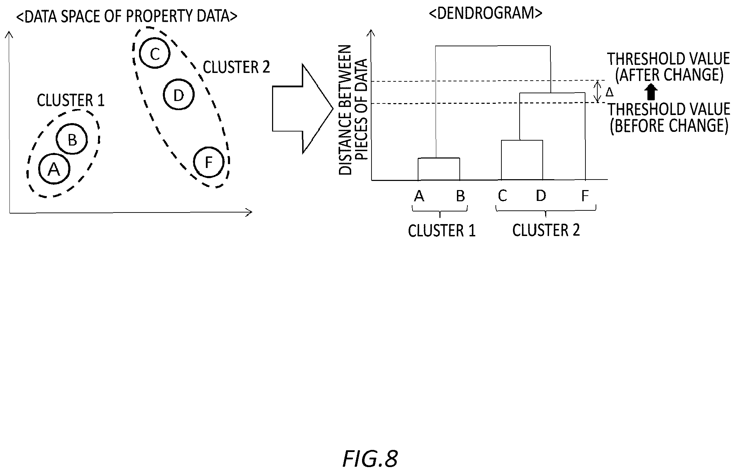

Fig. 8 shows examples of a data space of property data and a dendrogram after threshold value changes for crude oils A, B, C, D, and F. In Fig. 8, members having the same functions and configurations as those in Fig. 7 are denoted by the same signs and numerals, and the descriptions thereof will be omitted except for the following differences.

In Fig. 8, the threshold value is changed to be increased by the change width Δ. In this manner, as shown in the dendrogram, it can be seen that: the distance between pieces of data, between the crude oil F and the cluster 2 is short (less than the threshold value); and thus, the crude oil F is clustered in cluster 2. In this case, the model selection unit 130 may determine that the crude oil C and the crude oil D belonging to the cluster 2 have properties similar to that of the crude oil F. Then, the model selection unit 130 may select, as the target model when the crude oil F is used in the facility 10, the evaluation model 2 corresponding to the crude oil C and the crude oil D, that is, the cluster 2. According to this, the target model output unit 140 may output the evaluation model 2 as the target model.

It should be noted that Fig. 5 to Fig. 8 show the examples in which the hierarchical clustering is used when the model selection apparatus 100 clusters the property data; however, as described above, the model selection apparatus 100 may use the non-hierarchical clustering in clustering the property data. That is, the model selection unit 130 only needs to determine that the raw material belonging to the same cluster as that of the target raw material, as a result of clustering the property data, is similar to the target raw material, and the clustering method itself is not limited at all.

In the facility 10, in a case where the raw material which is identified as a plurality of raw materials depending on the production region, the production time, or the like, is used, when the evaluation model is generated for each raw material, the processing load increases and the time loss occurs. In contrast to this, the model selection apparatus 100 according to the present embodiment stores the plurality of evaluation models in association with the raw materials, and selects the target model for evaluating the state of the facility 10 based on the property data indicating the property of the target raw material, from among the plurality of evaluation models. In this manner, with the model selection apparatus 100 according to the present embodiment, the evaluation model selected based on the objective data from among the plurality of evaluation models, is provided as the target model for evaluating the facility 10, and thus, it is possible to omit the process of newly generating the evaluation model corresponding to the target raw material, which makes it possible to reduce the processing load and suppress a frequency of occurrence of the time loss.

In addition, the model selection apparatus 100 according to the present embodiment may select, as the target model, the evaluation model corresponding to the raw material with the property similar to that of the target raw material. In this manner, with the model selection apparatus 100 according to the present embodiment, it is possible to select, as the target model, the optimum evaluation model for evaluating the state of the facility 10 in light of the property of the raw material.

In particular, it is conceivable to select the target model based on the production region or the production time; however even when the distance between pieces of data for the production region or the production time is long, the properties of the raw materials may be similar to each other, and even when the distance between pieces of data for the production region or the production time is short, the properties of the raw materials may be greatly different from each other. With the model selection apparatus 100 according to the present embodiment, the target model is selected based on the similarity of the properties of the raw materials, and thus, even in such a case, it is possible to select, as the target model, the optimum evaluation model.

In addition, the model selection apparatus 100 according to the present embodiment may determine that the raw material belonging to the same cluster as that of the target raw material, as a result of clustering the property data, for example, in this way, has the property similar to that of the target raw material. In this manner, with the present embodiment, it is possible to determine the similarity of the properties of the raw materials, which is comparatively difficult to determine, based on a clear criterion regarding whether the raw materials belong to the same cluster.

In addition, when none of the raw materials belongs to the same cluster as that of the target raw material as a result of the hierarchical clustering of the property data, for example, in this way, the model selection apparatus 100 according to the present embodiment may change the threshold value between pieces of data, which is used for the hierarchical clustering. In this manner, with the model selection apparatus 100 according to the present embodiment, it is possible to group the target raw material into any of the existing clusters, and thus, it is possible to select any evaluation model as the target model from among the plurality of evaluation models. Accordingly, with the model selection apparatus 100 according to the present embodiment, it is possible to avoid an event in which the target model cannot be selected, which makes it possible to prevent a new evaluation model from being generated.

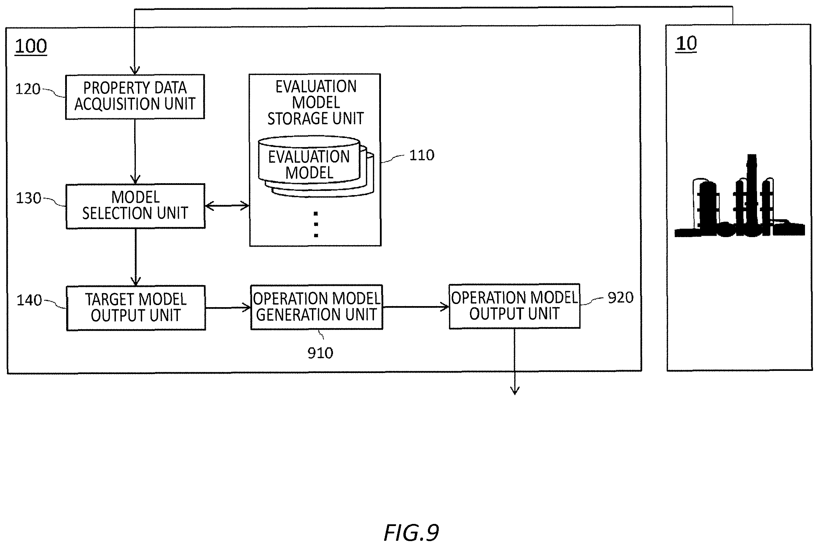

Fig. 9 shows an example of a block diagram of the model selection apparatus 100 according to a first modification example of the present embodiment, together with the facility 10. In Fig. 9, members having the same functions and configurations as those in Fig. 1 are denoted by the same signs and numerals, and the descriptions thereof will be omitted except for the following differences. The embodiment described above has shown, as an example, the case where the model selection apparatus 100, the operation model generation apparatus, and the control apparatus are respectively provided as independent separate apparatuses. However, these apparatuses may be provided as one apparatus in which some or all of them are integrated. In the present modification example, the model selection apparatus 100 provides the function of the operation model generation apparatus, in addition to the function of the model selection apparatus 100 according to the embodiment described above.

The model selection apparatus 100 according to the present modification example may further include an operation model generation unit 910 and an operation model output unit 920.

The operation model generation unit 910 is configured to generate the operation model that is configured to output the action in accordance with the state of the facility 10, by the reinforcement learning in which the output of the target model is set as at least a part of the reward. For example, the operation model generation unit 910 may generate the operation model, by acquiring the target model output by the target model output unit 150 and performing the reinforcement learning in which the output of the target model is set as at least a part of the reward.

Such an operation model may have, as an example, a data table that is constituted by a combination (S, A) of S representing a set of pieces of sampled state data and an action A taken under each state; and a weight W calculated by the reward. It should be noted that the output of the evaluation model may be used, as at least part of the reward for calculating such a weight W.

In generating such an operation model, the operation model generation unit 910 may acquire learning environment data indicating a state of a learning environment. At this time, when a simulator that simulates the operation in the facility 10 is used as the learning environment, the operation model generation unit 910 may acquire simulation data from the simulator as the learning environment data. However, the present invention is not limited to this. The actual facility 10 may be used as the learning environment. In this case, the operation model generation unit 910 may acquire the state data indicating the state of the facility 10 as the learning environment data.

Next, the operation model generation unit 910 may determine the action randomly or by using a known AI algorithm such as FKDPP which will be described below, and give the manipulated variable based on the action to the control target in the learning environment. The state of the learning environment is changed according to this.

Then, the operation model generation unit 910 may acquire the learning environment data again. This makes it possible for the operation model generation unit 910 to acquire the state of the learning environment after the change according to the manipulated variable being provided to the control target based on the determined action.

Then, the operation model generation unit 910 may calculate a reward value at least partially based on the output of the target model. As an example, according to the learning environment data indicating the state of the learning environment after the change, being input to the evaluation model, the index which is output by the target model may be calculated as the reward value, as it is.

The operation model generation unit 910 may update the operation model by repeating, multiple times, acquiring processing of the state in accordance with the determination of such an action, and then adding new sample data which has not been saved up to this point of time, to a new row in the data table, other than overwriting a value of a weight column in the data table. The operation model generation unit 910 can generate the operation model by repeating such update processing multiple times. For the generation itself of the operation model, any process may be used, and thus, a further description will be omitted here. The operation model generation unit 910 supplies the generated operation model to the operation model output unit 920.

The operation model output unit 920 outputs the operation model. For example, the operation model output unit 920 may output the operation model generated by the operation model generation unit 910, by transmitting the target model to another apparatus via the network. In particular, the operation model output unit 920 may transmit the generated operation model to the control apparatus. According to this, the control apparatus may control the control target provided in the facility 10 by using the operation model. However, the present invention is not limited to this. The operation model output unit 920 may output the generated operation model by writing the generated operation model to various memory devices, or may output the generated operation model by displaying the generated operation model on a monitor.

In this way, the model selection apparatus 100 according to the present modification example can also generate the operation model, by performing the reinforcement learning in which the output of the target model is set as at least a part of the reward. In this manner, with the model selection apparatus 100 according to the present modification example, it is possible to realize, by a single apparatus, the function of selecting the evaluation model and the function of generating the operation model. Accordingly, with the model selection apparatus 100 according to the present modification example, there is no need to exchange the evaluation model (the target model) between the model selection apparatus 100 and the operation model generation apparatus, and thus, it is possible to reduce a communication cost and time.

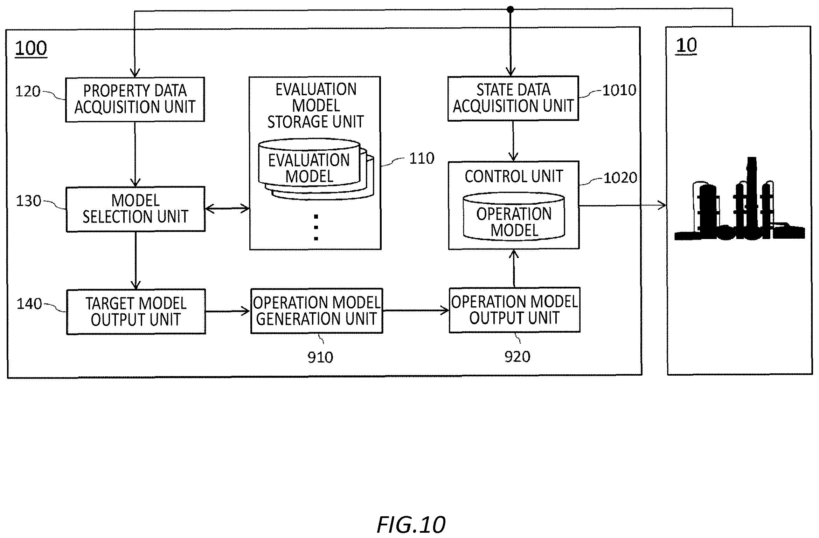

Fig. 10 shows an example of a block diagram of the model selection apparatus 100 according to a second modification example of the present embodiment, together with the facility 10. In Fig. 10, members having the same functions and configurations as those in Fig. 9 are denoted by the same signs and numerals, and the descriptions thereof will be omitted except for the following differences. In the first modification example, the case where the model selection apparatus 100 provides the function of the operation model generation apparatus is shown as an example. However, in the present modification example, the model selection apparatus 100 also provides the function of the control apparatus in addition to the function of the operation model generation apparatus.

The model selection apparatus 100 according to the present modification example may further include a state data acquisition unit 1010 and a control unit 1020. Then, the operation model output unit 920 may supply the generated operation model to the control unit 1020.

The state data acquisition unit 1010 acquires the state data indicating the state of the facility 10. For example, the state data acquisition unit 1010 may acquire various physical quantities measured in time series by various sensors provided in the facility 10, as the state data, from the facility 10 via the network. However, the present invention is not limited to this. The state data acquisition unit 1010 may acquire the state data via other means such as a user input or various memory devices, or may acquire the state data from another apparatus different from the facility 10. The state data acquisition unit 1010 supplies the acquired state data to the control unit 1020.

The control unit 1020 is configured to control the control target in the facility 10 by using the operation model. For example, the control unit 1020 may determine the action by a known AI algorithm such as the FKDPP. When such a kernel method is used, the control unit 1020 may generate a vector of a state S from a sensor value obtained by the acquired state data. Next, the control unit 1020 may generate, as an action determination table, a combination of the state S and all the actions that can be obtained. Then, the control unit 1020 may input the action determination table to the operation model. According to this, the operation model may perform a kernel calculation between each row of the action determination table, and each sample data of the data table in which the weight column is excluded, and calculate a distance to each piece of sample data. Next, the operation model may sequentially add a value obtained by multiplying the distance calculated for each sample data, by the value of each weight column, to calculate an expected reward value for each action. Then, the operation model may output the action of which the expected reward value is the highest among all the actions that can be obtained. The control unit 1020 may determine the action by selecting the action output by the operation model, for example, in this way.

Then, the control unit 1020 may give the control target the manipulated variable obtained by adding the determined action to a current value (for example, a current opening degree of the valve) of the control target. For example, in this way, the control unit 1020 can perform the AI control on the control target by using the generated operation model.

In this way, the model selection apparatus 100 according to the present modification example can control the control target by using the operation model generated by the reinforcement learning. In this manner, with the model selection apparatus 100 according to the present modification example, it is possible to realize, by a single apparatus, the function of selecting the evaluation model, the function of generating the operation model, and the function of controlling the control target. Accordingly, with the model selection apparatus 100 according to the present modification example, there is no need to exchange the evaluation model (the target model) between the model selection apparatus 100 and the operation model generation apparatus, and there is no need to exchange the operation model between the operation model generation apparatus and the control apparatus, as well, and thus, it is possible to reduce a communication cost and time.

So far, various embodiments that are possible have been described as examples. However, the embodiment described above may be modified or applied in various modes. For example, in the embodiment described above, the case where the model selection apparatus 100 changes, in a stepwise manner, the threshold value of the distance between pieces of data, which is used for the hierarchical clustering, until the target raw material is clustered into the existing cluster, is shown as an example. However, as the threshold value is changed, the similarity in property between the target raw material, and the raw material belonging to the existing cluster, gradually decreases. Accordingly, it is conceivable that the target model selected under such a circumstance is less appropriate for the target raw material, in comparison with the target model selected without changing the threshold value.

Therefore, in outputting the target model, the target model output unit 140 may provide a condition when the target model is selected, as well, by outputting information for identifying the threshold value used when the target model is selected, as well.

In addition, in the embodiment described above, the case where the model selection apparatus 100 necessarily causes the target raw material to be clustered into any of the existing clusters, is shown as an example. However, when none of the raw materials belongs to the same cluster as that of the target raw material as a result of the hierarchical clustering of the property data, for example, in this way, the model selection apparatus 100 may provide a message to that effect and end the flow without selecting the target model. In this case, the model selection apparatus 100 may omit the processing of changing the threshold value, or may limit the number of times the processing of changing the threshold value is executed. That is, the model selection apparatus 100 may execute the processing of changing the threshold value an upper limit number of times. Then, when none of the raw materials still belongs to the same cluster as that of the target raw material, a message to that effect may be provided for the flow to be ended without selecting the target model.

Various embodiments of the present invention may be described with reference to flowcharts and block diagrams, whose blocks may represent (1) steps of processes in which operations are executed or (2) sections of apparatuses responsible for executing operations. Certain steps and sections may be implemented by dedicated circuitry, programmable circuitry supplied with computer-readable instructions stored on computer-readable media, and/or processors supplied with computer-readable instructions stored on computer-readable media. Dedicated circuitry may include digital and/or analog hardware circuits, and may include integrated circuits (IC) and/or discrete circuits. The programmable circuitry may include a reconfigurable hardware circuit including logical AND, logical OR, logical XOR, logical NAND, logical NOR, and other logical operations, a memory element such as a flip-flop, a register, a field-programmable gate array (FPGA) and a programmable logic array (PLA), and the like.

A computer-readable medium may include any tangible device that can store instructions to be executed by a suitable device, and as a result, the computer-readable medium having instructions stored thereon includes an article of manufacture including instructions which can be executed to create means for performing operations specified in the flowcharts or block diagrams. Examples of the computer-readable medium may include an electronic storage medium, a magnetic storage medium, an optical storage medium, an electromagnetic storage medium, a semiconductor storage medium, and the like. More specific examples of the computer-readable medium may include a floppy (registered trademark) disk, a diskette, a hard disk, a random access memory (RAM), a read-only memory (ROM), an erasable programmable read-only memory (EPROM or flash memory), an electrically erasable programmable read-only memory (EEPROM), a static random access memory (SRAM), a compact disc read-only memory (CD-ROM), a digital versatile disc (DVD), a Blu-ray (registered trademark) disc, a memory stick, an integrated circuit card, and the like.

The computer-readable instruction may include: an assembler instruction, an instruction-set-architecture (ISA) instruction; a machine instruction; a machine dependent instruction; a microcode; a firmware instruction; state-setting data; or either a source code or an object code written in any combination of one or more programming languages, including an object oriented programming language such as Smalltalk (registered trademark), JAVA (registered trademark), C++, or the like, and a conventional procedural programming language such as a "C" programming language or a similar programming language.

Computer-readable instructions may be provided to a processor of a general purpose computer, special purpose computer, or other programmable data processing apparatuses, or to programmable circuitry, locally or via a local area network (LAN), wide area network (WAN) such as the Internet, or the like, to execute the computer-readable instructions to create means for performing operations specified in the flowcharts or block diagrams. Examples of the processor include a computer processor, a processing unit, a microprocessor, a digital signal processor, a controller, a microcontroller, or the like.

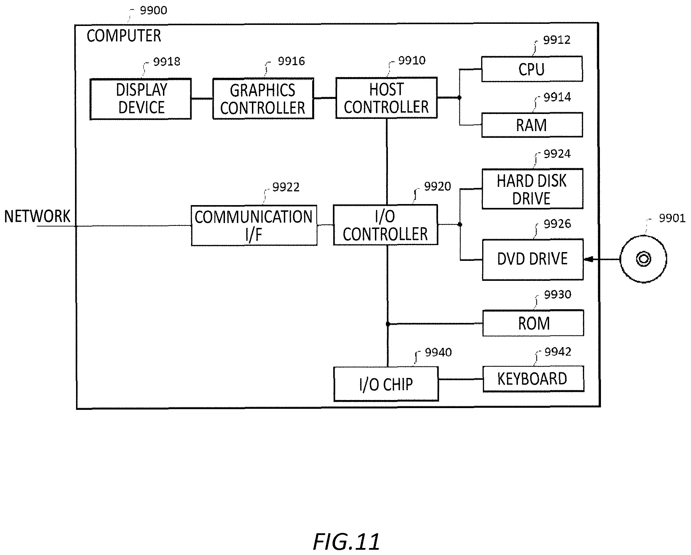

Fig. 11 shows an example of a computer 9900 in which a plurality of aspects of the present invention may be entirely or partially embodied. A program that is installed in the computer 9900 can cause the computer 9900 to function as operations associated with apparatuses according to the embodiments of the present invention or one or more sections of the apparatuses, or can cause the computer 9900 to execute the operations or the one or more sections thereof, and/or can cause the computer 9900 to execute processes according to the embodiments of the present invention or steps of the processes. Such a program may be executed by a CPU 9912 so as to cause the computer 9900 to execute certain operations associated with some or all of the flowcharts and the blocks in the block diagrams described herein.

The computer 9900 according to the present embodiment includes the CPU 9912, a RAM 9914, a graphics controller 9916, and a display device 9918, which are mutually connected by a host controller 9910. The computer 9900 further includes input/output units such as a communication interface 9922, a hard disk drive 9924, a DVD drive 9926, and an IC card drive, which are connected to the host controller 9910 via an input/output controller 9920. The computer also includes legacy input/output units such as a ROM 9930 and a keyboard 9942, which are connected to the input/output controller 9920 via an input/output chip 9940.

The CPU 9912 operates according to programs stored in the ROM 9930 and the RAM 9914, thereby controlling each unit. The graphics controller 9916 acquires image data generated by the CPU 9912 on a frame buffer or the like provided in the RAM 9914 or in itself, and causes the image data to be displayed on the display device 9918.

The communication interface 9922 communicates with other electronic devices via a network. The hard disk drive 9924 stores programs and data that are used by the CPU 9912 within the computer 9900. The DVD drive 9926 reads programs or data from a DVD-ROM 9901, and provides the hard disk drive 9924 with the programs or the data via the RAM 9914. The IC card drive reads the programs and the data from the IC card, and/or writes the programs and the data to the IC card.

The ROM 9930 stores therein a boot program or the like executed by the computer 9900 at the time of activation, and/or a program depending on the hardware of the computer 9900. The input/output chip 9940 may also connect various input/output units, via a parallel port, a serial port, a keyboard port, a mouse port, or the like, to the input/output controller 9920.

A program is provided by a computer-readable medium such as the DVD-ROM 9901 or the IC card. The program is read from the computer-readable medium, installed into the hard disk drive 9924, the RAM 9914, or the ROM 9930, which are also examples of the computer-readable medium, and executed by the CPU 9912. The information processing written in these programs is read into the computer 9900, resulting in cooperation between a program and the above-mentioned various types of hardware resources. An apparatus or a method may be constituted by actualizing the operation or processing of information in accordance with the usage of the computer 9900.

For example, when communication is performed between the computer 9900 and an external device, the CPU 9912 may execute a communication program loaded onto the RAM 9914 to instruct the communication interface 9922 to process the communication, based on the processing written in the communication program. The communication interface 9922, under control of the CPU 9912, reads transmission data stored on a transmission buffer region provided in a recording medium such as the RAM 9914, the hard disk drive 9924, the DVD-ROM 9901, or the IC card, and transmits the read transmission data to a network or writes reception data received from a network to a reception buffer region or the like provided on the recording medium.

Also, the CPU 9912 may cause all or a necessary portion of a file or a database to be read into the RAM 9914, the file or the database having been stored in an external recording medium such as the hard disk drive 9924, the DVD drive 9926 (the DVD-ROM 9901), the IC card, etc., and perform various types of processing on the data on the RAM 9914. The CPU 9912 then writes back the processed data to the external recording medium.

Various types of information such as various types of programs, data, tables, and databases may be stored in a recording medium and subjected to information processing. The CPU 9912 may perform various types of processing on the data read from the RAM 9914, which includes various types of operations, information processing, condition judging, conditional branch, unconditional branch, search/replacement of information, etc., as described throughout the present disclosure and designated by an instruction sequence of programs, to write the result back to the RAM 9914. Also, the CPU 9912 may search for information in a file, a database, etc., in the recording medium. For example, when a plurality of entries, each having an attribute value of a first attribute associated with an attribute value of a second attribute, are stored in the recording medium, the CPU 9912 may search for an entry matching the condition whose attribute value of the first attribute is designated, from among the plurality of entries, and read the attribute value of the second attribute stored in the entry, thereby obtaining the attribute value of the second attribute associated with the first attribute satisfying the predetermined condition.

The above described program or software modules may be stored in the computer-readable medium on or near the computer 9900. Also, a recording medium such as a hard disk or a RAM provided in a server system connected to a dedicated communication network or the Internet can be used as the computer-readable medium, thereby providing the program to the computer 9900 via the network.

While the present invention has been described with the embodiments, the technical scope of the present invention is not limited to the above-described embodiments. It is apparent to persons skilled in the art that various alterations and improvements can be added to the above-described embodiments. It is also apparent from the description of the claims that the embodiments to which such alterations or improvements are made can be included in the technical scope of the present invention.

The operations, procedures, steps, and stages of each process performed by an apparatus, system, program, and method shown in the claims, specification, or drawings can be performed in any order as long as the order is not indicated by "prior to," "before," or the like and as long as the output from a previous process is not used in a later process. Even if the process flow is described using phrases such as "first" or "next" in the claims, specification, or drawings, it does not necessarily mean that the process must be performed in this order.

### EXPLANATION OF REFERENCES

10 facility
100 model selection apparatus
110 evaluation model storage unit
120 property data acquisition unit
130 model selection unit
140 target model output unit
910 operation model generation unit
920 operation model output unit
1010 state data acquisition unit
1020 control unit
9900 computer
9901 DVD-ROM
9910 host controller
9912 CPU
9914 RAM
9916 graphics controller
9918 display device
9920 input/output controller
9922 communication interface
9924 hard disk drive
9926 DVD drive
9930 ROM
9940 input/output chip
9942 keyboard.

## Claims

1. A model selection apparatus comprising:
an evaluation model storage unit configured to store each of a plurality of evaluation models capable of outputting an index for evaluating a state of a facility that is configured to manufacture a product from a raw material, in association with the raw material;
a property data acquisition unit configured to acquire property data indicating a property of the raw material which is used in the facility;
a model selection unit configured to select a target model for evaluating the state of the facility based on the property data, from among the plurality of evaluation models, when a target raw material in the facility is used; and
a target model output unit configured to output the target model.

2. The model selection apparatus according to claim 1, wherein the model selection unit is configured to select, as the target model, an evaluation model corresponding to a raw material with a property similar to that of the target raw material, from among the plurality of evaluation models.

3. The model selection apparatus according to claim 2, wherein the model selection unit is configured to determine that a raw material belonging to a same cluster as that of the target raw material, as a result of clustering the property data, has a property similar to that of the target raw material.

4. The model selection apparatus according to claim 3, wherein the model selection unit is configured to change, when none of raw materials belongs to the same cluster as that of the target raw material as a result of hierarchical clustering of the property data, a threshold value of a distance between pieces of data, which is used for the hierarchical clustering.

5. The model selection apparatus according to any one of claims 1 to 4, wherein the raw material includes at least crude oil.

6. The model selection apparatus according to claim 5, wherein the property data has data indicating at least any of a chemical property or a physical property of the crude oil.

7. The model selection apparatus according to claim 6, wherein the chemical property includes a content of at least any of carbon, hydrogen, sulfur, nitrogen, oxygen, or metal.

8. The model selection apparatus according to claim 6, wherein the chemical property includes a type of a molecular structure of a hydrocarbon.

9. The model selection apparatus according to claim 6, wherein the physical property includes at least any of specific gravity, vapor pressure, kinematic viscosity, or pour point.

10. The model selection apparatus according to any one of claims 1 to 4, wherein the raw material includes at least any of a fossil fuel or water.

11. The model selection apparatus according to any one of claims 1 to 4, further comprising an operation model generation unit configured to generate an operation model that is configured to output an action in accordance with the state of the facility, by reinforcement learning in which an output of the target model is set as at least a part of a reward.

12. The model selection apparatus according to claim 11, further comprising a control unit configured to control a control target in the facility by using the operation model.

13. A model selection method that is executed by a computer, the model selection method comprising: by the computer,
storing each of a plurality of evaluation models capable of outputting an index for evaluating a state of a facility that is configured to manufacture a product from a raw material, in association with the raw material;
acquiring property data indicating a property of the raw material which is used in the facility;
selecting a target model for evaluating the state of the facility based on the property data, from among the plurality of evaluation models, when a target raw material in the facility is used; and
outputting the target model.

14. A model selection program that is executed by a computer and that causes the computer to function as:
an evaluation model storage unit configured to store each of a plurality of evaluation models capable of outputting an index for evaluating a state of a facility that is configured to manufacture a product from a raw material, in association with the raw material;
a property data acquisition unit configured to acquire property data indicating a property of the raw material which is used in the facility;
a model selection unit configured to select a target model for evaluating the state of the facility based on the property data, from among the plurality of evaluation models, when a target raw material in the facility is used; and
a target model output unit configured to output the target model.
